# EUROPÄISCHE PATENTSCHRIFT

(11) **EP 0 707 859 B1**
(45) Veröffentlichungstag und Bekanntmachung des Hinweises auf die Patenterteilung: **15.01.2003**
(21) Anmeldenummer: 95109190.9
(22) Anmeldetag: 14.06.1995
(51) Int. Cl.: A61M 5/178, A61M 5/32, A61M 5/34

(54) **Behältnis zur Lagerung und Verabreichung von Injektions-, Infusions- und Diagnostikpräparaten**
Containers for storage and administration of preparations suited for injection, infusion or diagnostic purpose
Récipient pour le stockage et l'administration de préparations d'injection, d'infusion et de diagnostic

(30) Priorität: 28.09.1994 DE 4434644
(43) Veröffentlichungstag der Anmeldung: 24.04.1996
(73) Patentinhaber: Schott Glas, 55122 Mainz (DE); CARL-ZEISS-STIFTUNG trading as Schott Glas, 55122 Mainz (DE)
(72) Erfinder: Reinhard, Michael, D-55270 Ober-Olm (DE); Spallek, Michael, D-55218 Ingelheim (DE)
(74) Vertreter: Fuchs, Jürgen H., Dr.-Ing.

(56) Entgegenhaltungen:
- EP-A- 0 519 240
- EP-A- 0 567 186
- WO-A-90/00073
- WO-A-92/04927
- DE-A- 3 916 101
- GB-A- 2 249 727
- US-A- 4 723 945

## Beschreibung

Die Erfindung betrifft ein Behältnis für Injektions-, Infusions- und Diagnostikpräparate mit einem Füllvolumen im Bereich zwischen 10 und 200 ml zur langfristigen Lagerung sowie zur direkten Verabreichung der Präparate, bestehend aus einem Glaszylinder
- bei dem die Abmessungen hinsichtlich des Verhältnisses der Gesamthöhe zum äußeren Durchmesser auf die Abmessungen einer handelsüblichen Spritze abgestellt sind (Verhältnis größer als 2,5:1),
- an dem kopfseitig ein konvergierendes Glas-Kopfteil angeformt ist, das ein für die Verabreichung in eine Kanüle oder Infusionsleitung öffenbares Verschlußteil besitzt,
- der auf der anderen Seite (Fußseite) eine Kunststoff-Griffleiste aufweist, die mit einem zylindrischen Fortsatz an der Innenwand des Glaszylinders mit einem Schnappverschluß befestigt ist, und
- der mit einem als Kolben dienenden Stopfen, der eine Verbindungsstelle für die Befestigung einer als Spritzenstempel dienenden Kolbenstange aufweist, verschlossen ist.

Für Zwecke der Verabreichung von Injektions-, Infusions- und Diagnostikpräparaten kommen Glasbehältnisse zur Lagerung dieser Präparate (gemäß DIN ISO 8362, Teil 1-4) fertig abgefüllt in den Verkehr, die mit einem Gummistopfen verschlossen sind. Dieser Gummistopfen ist so ausgebildet, daß er selbstabdichtend von der Kanüle einer Spritze, insbesondere einer heute üblichen Kunststoff-Einmalspritze (nach DIN 13 098), mehrfach durchstechbar ist, wodurch die Spritzen aus dem Vorratsbehältnis heraus mehrfach aufziehbar sind.

Es ist auch bekannt, vorgenannte Lagerbehältnisse so auszubilden bzw. durch Anbringen von Zusatzteilen so auszurüsten, daß sie die Funktion einer Spritze erhalten und aus ihnen heraus auch unmittelbar die Verabreichung des jeweiligen Präparates erfolgen kann. Dadurch entfällt die Umfüllung aus einem

Behältnis in die heute üblichen Kunststoffspritzen, wodurch der Arbeitsaufwand, die Verwechslungsgefahr und die Kontaminationsgefahr erheblich reduziert werden. Außerdem stellt die direkte Verabreichung aus dem Behältnis unter dem gesamtheitlichen Aspekt der Gesamtkosten der Arzneimittelverabreichung die mit Abstand kostengünstigste Möglichkeit dar.

Aus der EP O 298 585 A1 ist ein derartiges Behältnis bekannt, das ein Füllvolumen zwischen 20 und 100 ml aufweist und das vom Prinzip her ein Fläschchen mit variablem Boden ist. Es entspricht mit einem Verhältnis von Gesamthöhe zu Durchmesser von nicht über 2,5:1 typischen Injektionsfläschchen, die gemäß DIN ISO 8362, Teil 1, entsprechende Maßverhältnisse von weniger als 2,9:1 aufweisen. Anstelle eines Glasbodens weist dieses Fläschchen einen Gummistopfen auf, an dem ein Kolbenstempel befestigt werden kann. Die Fläschchenmündung ist als Bördelrand ähnlich DIN ISO 8362, Teil 1, ausgebildet, die Fläschchenöffnung ist mit einem Gummistopfen ähnlich ISO 8362, Teil 2, verschlossen, und eine Aluminiumbördelkappe ähnlich DIN ISO 8362, Teil 3, sichert den Gummistopfen in seiner Position. Zur Aktivierung muß der mittlere Bereich des Gummistopfens zugänglich sein. Bei Bördelkappen, die nicht von vornherein in der Mitte eine Öffnung aufweisen, sondern verschlossen sind, wird der mittlere Bereich zuerst entfernt. Hierfür ist ein Öffnungsmechanismus mit Sollbruchstellen vorgesehen. Im nächsten Schritt muß dann der freigelegte Bereich des Gummis desinfiziert werden. Anschließend erfolgt die eigentliche Aktivierung des Fläschchens, indem ein Aktivierungsaufsatz über die Bördelkappe geschoben wird. Dieser Aktivierungsaufsatz ist zentrisch auf der dem Gummi zugewandten Seite mit einer Nadel ausgerüstet, die den Gummi beim Aufschieben des Aktivierungsaufsatzes durchsticht und somit einen Zugang zum Präparat schafft. Für Injektionszwecke kann die Nadel eine beidseitige Nadel sein, d.h. die eine Seite dient zum Durchstoßen des Gummis zur Aktivierung, während die andere, längere Seite der Nadel die Funktion der Injektionsnadel hat. Für andere Zwecke ist der Aktivierungsaufsatz nur auf der einen Seite mit einer Nadel versehen, währenddem die andere Seite als verriegelbare Kegelverbindung (nach DIN 13090, Teil 2) ausgeführt ist. An diese Verbindung kann wahlweise eine Kanüle oder eine Infusionsleitung angeschlossen werden.

Nachteilig an diesem bekannten Behältnis ist, daß erst durch das Aufschieben des Aktivierungsaufsatzes das Fläschchen mit variablem Boden von seiner Funktion her zu einer Spritze wird, die jedoch in ihren Abmessungen mit einem Verhältnis von Gesamthöhe zu äußerem Durchmesser von nicht über 2,5:1 in übliche Spritzenpumpen nicht einsetzbar ist, da diese für marktübliche, nicht vorfüllbare Einmalspritzen aus Kunststoffen (DIN ISO 7886) konzipiert sind.

Die Einsetzbarkeit in Spritzenpumpen ist jedoch ein wesentliches Erfordernis, denn bei vielen medizinischen Anwendungen erfolgt die Verabreichung des Präparates mittels einer Spritzenpumpe, in die die Spritze eingelegt wird. Über einen einstellbaren Vorschub wird der Kolbenstempel der Spritze bewegt, währenddem der Spritzenkörper fixiert ist, so daß das Präparat in eine Infusionsleitung verdrängt wird. Im Gebrauch können hohe Kräfte von mehr als 50 N auf die Griffleiste einer Spritze übertragen werden, so daß die Abziehkräfte für die Griffleiste von für Spritzenpumpen geeigneten Spritzen deutlich über 50 N liegen müssen.

Eine Übersicht über heute marktübliche Spritzenpumpen ist im American Journal of Hospital Pharmacy, Band 48, Oktober 1991, Suppl. 1, Seiten S36-S51 (Herausgeber American Society of Hospital Pharmacists Inc.) gegeben.

Ein weiterer Nachteil dieses bekannten Behältnis-Systems liegt darin, daß zusätzlich zum Behältnis noch ein Aktivierungsaufsatz erforderlich ist. Das Reinigen des Gummistopfens in dem Bereich, in dem die Aktivierungsnadel durch den Stopfen tritt sowie das Aufschieben des Aktivierungsaufsatzes bedeuten einen nicht unerheblichen Handhabungsaufwand.

Stets müssen ferner Gummistopfen durchstoßen werden, wobei kleinste Gummipartikel erzeugt werden können (DIN 58367), die aus medizinischer Sicht problematisch sein können.

Ein weiterer Nachteil dieses bekannten Systems besteht in der notwendigen Verwendung von Aluminiumbördelkappen, was zum einen Abriebpartikel bei der Abfüllung erzeugt, zum anderen eine Materialtrennung und Wiederverwertung im nicht-pharmazeutischen Bereich erschwert.

Aus der DE 39 24 830 A1 ist ferner ein zu einer Spritze konfektionierbarer Spritzenzylinder bekannt, der aus einem Glasrohr besteht, das an einem Ende einen verschiebbaren Spritzenkolben mit Fingerauflage sowie am anderen, kanülenseitigen Ende, ein Verschlußteil in Form eines Durchstech-Gummistopfens mit Kopfteil aufweist. Dieser Spritzenzylinder zeichnet sich durch einen komplizierten Aufbau aus zahlreichen Teilen aus; bei der Anwendung als Spritze müssen Gummiteile durchstoßen werden, was zu den eingangs erläuterten Nachteilen führt. Die Anbringung von Fingerauflage und Kopfteil erhöht den Zylinderdurchmesser auf längeren Bereichen und verhindert somit mit Nachteil die Möglichkeit der Verwendung solcher Spritzen in Spritzenpumpen. Gleiches gilt für die entsprechend aufgebauten Spritzen gemäß der DE 39 16 101 A1 und WO 91/01152.

Durch die GB 2 249 727 A ist das eingangs bezeichnete Behältnis für Injektions-, Infusions- und Diagnostikpräparate bekanntgeworden. Von diesem bekannten Behältnis geht die Erfindung aus.

Bei einem derartigen Behältnis kommt es wesentlich auf die Ausbildung des Spritzenkopfes und dessen Verschluß während der Lagerung des Behältnisses bzw. dessen Anschließbarkeit an eine Kanüle oder Infusionsleitung an. Im bekannten Fall mündet das konvergierende Glas-Kopfteil in ein zylindrisches Austrittsteil mit kleinerem Querschnitt als das Behältnis selbst und mit einem wulstförmigen Rand. Es ist die übliche Ausbildung des Halses bei konventionellen Injektionsfläschchen. Dieses Austrittsteil ist mit einem Stopfen verschließbar, der in der Praxis üblicherweise aus einem Gummimaterial besteht. Dieser Stopfen hat - ganz entsprechend den konventionellen Injektionsfläschchen - eine flache Kopffläche. Der Stopfen samt dem wulstförmigen Rand des Austrittsteiles wird umfaßt von einer Aluminium-Kappe, die auch ein Kunststoff-Kuppelteil (Adapter) mit einem Flansch und einem Hohlzylinderteil, das die Kappe durchdringt und das ein Gewinde zur Aufnahme eines Nadeleinsatzes besitzt, umschließt. Der Hohlzylinder ist dabei bodenseitig durch ein dünnes Diaphragma abgeschlossen.

Alternativ kann dabei das Kuppelteil lediglich aus einem zylindrischen, einstückig mit der Kappe verbundenen Fortsatz mit dem Gewindeanschluß bestehen. Anstelle des Nadeleinsatzes kann im Alternativfall unmittelbar eine beidseitige Nadel in dem Fortsatz aufgenommen werden.

Durch die US 4,723,945 ist eine Spritze bekannt geworden, in deren Spritzenzylinder ein vorgefülltes Medizinfläschchen eingesetzt und mit mittels einer verriegelbaren Kegelverbindung dort befestigt ist.

Der Erfindung liegt die Aufgabe zugrunde, ein Behältnis der eingangs bezeichneten Art zu schaffen, das für die Verabreichung des Präparates ein Minimum an Handhabungsaufwand erfordert, das die Verwendung einer heute marktüblichen Spritzenpumpe zur Verabreichung ermöglicht, das kein Durchstoßen von Gummiteilen erfordert, das keine Metallkomponenten enthält und das besonders kostengünstig herzustellen ist.

Diese Aufgabe wird erfindungsgemäß dadurch gelöst, daß
- an dem konvergierenden Glas-Kopfteil ein als Spritzenkopf ausgebildeter Anschlußkonus einer Kegelverbindung einstückig angeformt ist,
- zur Verriegelung der Kegelverbindung ein separates Gewindeteil aus Kunststoff zentrisch zum Anschlußkonus an dessen Außenmantel befestigt ist,
- das Gewindeteil zur Aufnahme einer Verschlußkappe, mittels der der Anschlußkonus zwecks Lagerung des Behältnisinhaltes über die Kegelverbindung verschließbar ist, und zur Aufnahme der Kanüle oder Infusionsleitung über die Kegelverbindung nach vorhergehender Abnahme der Verschlußkappe zwecks direkter Verabreichung ausgebildet ist, sowie auf der der Befestigungsstelle am Außenmantel gegenüberliegenden Seite eine Sicherungskappe aufweist, wobei die Verbindungsstelle zwischen dem separaten Gewindeteil und der Sicherungskappe als Sollbruchstelle ausgebildet ist,
- die Griffleiste so am Glaszylinder befestigt ist, daß sie Abziehkräften von mindestens 100 N widersteht, indem der Glaszylinder (1) im Bereich des eingebrachten zylindrischen Fortsatzes (12) der Griffleiste (13) eine Hinterschneidung (10) und der Fortsatz eine dazu komplementäre, den Schnappverschluß bildende Hinterschneidung aufweist, und in den zylindrischen Fortsatz (12) der Griffleiste (13) ein Sicherungsring (13 b) eingeschoben ist und dort mittels einer Schnappverbindung gehaltert ist.

Dieses Behältnis hat den Vorteil, daß für die Verabreichung des Präparates auf einfache Weise lediglich die Verschlußkappe entfernt werden muß, und dann sofort eine Infusionsleitung oder eine Kanüle angeschlossen werden kann. Die Sicherungskappe mit Soll-Bruchstelle dient dabei einmal als Sicherung gegen unbeabsichtigtes Abziehen bzw. Lösen der Verschlußkappe und stellt zum anderen auch einen Originalitätsverschluß dar. Von besonderem Vorteil ist dabei auch, daß bei der Aufnahme der Kanüle oder Infusionsleitung zwecks direkter Verabreichung des Behältnisinhaltes, keine Elastomerteile, d.h. Gummistopfen, durchstochen werden müssen.

Zudem ermöglicht die geringe Anzahl von Teilen eine kostengünstige Herstellung unter Vermeidung von Aluminiumbördelkappen mit dem Nachteil der Abriebpartikel und der Entsorgung.

Die EP 0 567 186 zeigt eine übliche Spritze aus Kunststoff, die einen konusförmigen Spritzenkopf für eine Kegelverbindung besitzt, der zu Lagerzwecken mit einer entfernbaren Kappe versehen ist. Diese Spritze ist jedoch eine andere Pharmaverpackung als das Injektionsfläschchen aus Glas, von dem die Erfindung ausgeht.

Weiterhin sind aus der EP 0 382 126 A2 sogenannte vorgefüllte Glasfertigspritzen im Füllvolumenbereich kleiner, gleich 20 ml, bekannt, deren Spritzenzylinder und Kopfbereich, inklusive nicht verriegelbarer Kegelverbindung, aus Glas bestehen. Auch die Griffleiste besteht aus Glas, sie ist direkt an den Spritzenzylinder angeformt. Ähnlich gestaltet sind die aus der US-A 3 865 236 und DE 29 39 180 C2 bekannten Spritzen. Das Hauptanwendungsgebiet dieser Spritzen ist die Injektion. Für Sonderanwendungen gibt es auch Glasfertigspritzen, bei denen eine verriegelbare Kegelverbindung konzentrisch zum Anschlußkonus angebracht ist.

Von ihrer Gestaltung her eignen sich diese Spritzen aber nicht für die Verwendung in einer üblichen Spritzenpumpe.

Gemäß einer Ausgestaltung der Erfindung, bei der der Anschlußkonus auf seiner konischen, äußeren Mantelfläche in einem Bereich, der mehr als 5 mm von seiner Spritze entfernt liegt, eine Eindrehung aufweist, und das separate Gewindeteil der verriegelbaren Kegelverbindung unter Bildung einer formschlüssigen Schnapp-Verbindung in diese Eindrehung eingesetzt ist, ergibt sich der Vorteil, daß die Befestigung des losen Gewindeteils der verriegelbaren Kegelverbindung aus Kunststoff durch Einschnappen in eine Eindrehung ein hohes Maß an Sicherheit gegen Lösen der Verbindung bietet.

Bei einer weiteren Ausführungsform, bei der der Spritzenkopf im Übergangsbereich zwischen Anschlußkonus und Spritzenzylinder die Eindrehung aufweist, d.h. mit einem größeren Durchmesser der Eindrehung, wird die Stabilität der Befestigung der verriegelbaren Kegelverbindung verbessert und gleichzeitig die mechanische Festigkeit des Behältnisses am Auslauf des Anschlußkonus erhöht.

Anstelle einer Befestigung des separaten Gewindeteiles mittels einer Schnappverbindung kann das Gewindeteil bei beiden vorgenannten Ausführungsformen auf den Anschlußkonus bzw. im Übergangsbereich zum Spritzenzylinder aufgeklebt sein. Ein derartiges Behältnis hat den Vorteil, daß auf die Herstellung einer Eindrehung auf Glasmaterial verzichtet werden kann.

Für die Aufnahme der Verschlußkappe im separaten Gewindeteil stehen dem Fachmann eine Reihe von Möglichkeiten zur Verfügung. Im einfachsten Fall besitzt das separate Gewindeteil ein Innengewinde, in welches die Verschlußkappe mit einem Außengewinde einschraubbar ist, wobei die Verschlußkappe eine Auskleidung oder Einlage aus elastischem Material aufweist, die dichtend an der konischen äußeren Mantelfläche und/oder an der Stirnfläche des Anschlußkonus anliegt.

Eine geringe axiale Ausdehnung im Kappenbereich erzielt man, wenn nach einer weiteren Ausgestaltung der Erfindung das separate Gewindeteil der verriegelbaren Kegelverbindung aus Kunststoff ein Außengewinde aufweist, auf das eine deckelartige Verschlußkappe mit einem Innengewinde aufgeschraubt ist, in die eine Auskleidung oder Einlage aus elastischem Material eingebracht ist, die dichtend an der konischen, äußeren Mantelfläche und/oder an der Stirnfläche des Anschlußkonus anliegt.

Eine weitere Möglichkeit der Aufbringung der Verschlußkappe, die leicht zu handhaben und kostengünstig herzustellen ist, besteht darin, daß die Verschlußkappe aus Gummi besteht, die Innenkontur der Verschlußkappe eine Kontur entsprechend der Außenkontur des Anschlußkonus aufweist, wobei die Innenkontur der Verschlußkappe in bezug auf die Außenkontur des Anschlußkonus vom Durchmesser her Untermaß aufweist, die Verschlußkappe auf den Anschlußkonus aufgeschoben oder aufgedreht ist, wodurch die innere Mantelfläche der Verschlußkappe dichtend an der konischen, äußeren Mantelfläche des Anschlußkonus anliegt.

Wenn man das Behältnis nach der Erfindung in der Weise weiter ausgestaltet, daß der kleinste Innendurchmesser des separaten Gewindeteiles der verriegelbaren Kegelverbindung, einschließlich Schnappverbindungsteil bzw. Klebefläche größer ist als der größte Außendurchmesser der Verschlußkappe, erhält man einen wesentlichen Vorteil beim Füllen der Spritze. Zuerst wird die Spritze im Kopfbereich durch Aufschieben der Verschlußkappe auf den Anschlußkonus verschlossen. Der kleinste Innendurchmesser der verriegelbaren Kegelverbindung ist so groß, daß sie noch nachträglich über die Verschlußkappe hinweg geführt und in der Eindrehung bzw. auf dem Klebesitz befestigt werden kann. Dies bedeutet, daß die verriegelbare Kegelverbindung im Falle einer aseptischen Abfüllung nicht in den Sterilbereich eingebracht werden muß, was deutliche Handhabungs- und Kostenvorteile bei der Abfüllung bringt.

Die spezielle Gestaltung einer Verschlußkappe, die im unteren Bereich des Kappenhemdes auf der innenliegenden Fläche ein oder mehrere axiale Nuten aufweist, ermöglicht das Lyophilisieren in der Spritze mit bereits aufgesetzter Kappe. Zuerst wird die Verschlußkappe voll auf den Anschlußkonus aufgeschoben und eine Dichtwirkung erzielt. Die Spritze wird dann über das offene zylindrische Ende des Spritzenzylinders gefüllt. Anschlußkonus und Verschlußkappe befinden sich dabei unten. Nach der Füllung wird der Kolbenstopfen gesetzt, die Spritze gedreht und die Verschlußkappe soweit angehoben, daß sie einerseits noch fest auf dem Anschlußkonus sitzt, andererseits aber über die im unteren Bereich des Kappenhemdes auf der innenliegenden Fläche angebrachten axialen Nuten eine Verbindung zwischen dem Spritzeninnenraum und der Umgebung hergestellt wird. Nun kann die Lyophilisierung erfolgen und durch erneutes vollständiges Aufschieben der Verschlußkappe noch im Lyophilisator kann das Behältnis fertig verschlossen werden.

Weitere ausgestaltende Merkmale und Vorteile der Erfindung ergeben sich anhand der in den Zeichnungen dargestellten Ausführungsbeispiele.

Es zeigen:
- Fig. 1: einen Längsschnitt durch eine erste Ausführungsform eines erfindungsgemäßen Behältnisses mit Ausschnittvergrößerungen gemäß den Fig. 1a und 1b; die Fig. 1c zeigt dabei, in der Darstellung der Fig. 1b, eine besondere Ausführungsform mit eingeschobenem Sicherheitsring.
- Fig. 2: eine zweite Ausführungsform eines Spritzenkopfes des Behältnisses nach Fig. 1 im Längsschnitt,
- Fig. 3: eine dritte Ausführungsform eines Spritzenkopfes des Behältnisses nach Fig. 1 im Längsschnitt,
- Fig. 4: eine vierte Ausführungsform des Spritzenkopfes des erfindungsgemäßen Behältnisses nach Fig. 1 im Längsschnitt und zwar im Teil
a) im geöffneten Zustand für die Lyophilisation
b) im geschlossenen Zustand.

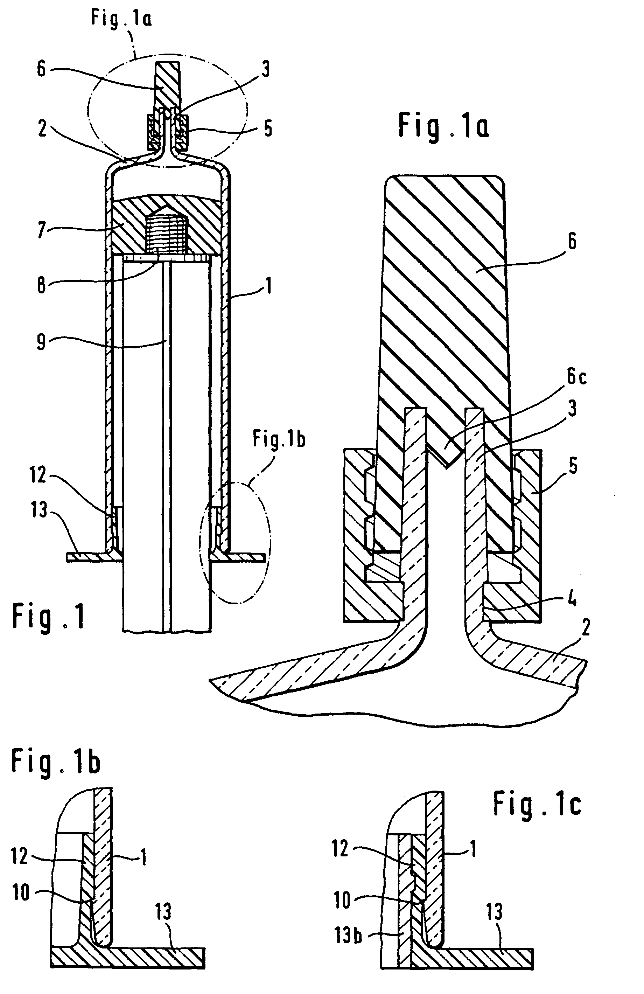

Die Figur 1 zeigt ein erfindungsgemäßes Behältnis für Injektions-Infusionsund Diagnostikpräparaten mit einem Füllvolumen im Bereich zwischen 10 und 200 ml zur langfristigen Lagerung sowie zur direkten Verabreichung der eingefüllten Präparate. Das Behältnis ist als Spritze konfektioniert, wobei die Figur 1 die Spritze im geschlossenen Zustand zeigt. Die Abmessungen des Behältnisses sind daher hinsichtlich des Verhältnisses der Gesamthöhe zum äußeren Durchmesser auf die Abmessungen einer handelsüblichen Spritze abgestellt, d.h. das Verhältnis ist größer als 2,5:1.

Das Behältnis besteht aus einem Glaszylinder 1, dem Spritzenzylinder, an dem kopfseitig ein konvergierendes Glaskopfteil 2 mit einem Glas-Anschlußkonus 3, der Spritzenkopf, einstückig angeformt ist, wie besonders gut aus der Ausschnittsvergrößerung gemäß Figur 1a zu erkennen ist. Der Anschlußkonus 3 weist eine Eindrehung (Nut) 4 auf, in die ein separates Gewindeteil 5 einer verriegelbaren Kegelverbindung aus Kunststoff konzentrisch zum Anschlußkonus unter Bildung einer formschlüssigen Schnappverbindung verdrehbar eingesetzt ist. Das Gewindeteil 5 nimmt eine Verschlußkappe 6, vorzugsweise eine Gummikappe, auf, mittels der der Anschlußkonus 3 durch Verdrehen des Gewindeteiles 5 unter Bildung einer Kegelverbindung zur Verschlußkappe 6 dicht verschließbar bzw. verriegelt ist. Die Innenkontur der Verschlußkappe 6 weist eine Kontur entsprechend der Außenkontur des Anschlußkonus 3 auf, jedoch mit einem Untermaß in bezug auf den Durchmesser. Nachdem die Verschlußkappe auf den Anschlußkonus 3 aufgeschoben oder aufgedreht ist, liegt dann die innere Mantelfläche der Verschlußkappe dichtend an der konischen, äußeren Mantelfläche des Anschlußkonus 3 an. Die Verschlußkappe weist ferner eine Auskleidung 6c aus elastischem Material auf, die dichtend an der konischen äußeren Mantelfläche und an der Stirnfläche des Anschlußkonus 3 anliegt.

Nach Aufschrauben des Gewindeteiles 5 kann die Verschlußkappe 6 abgenommen und eine Injektionskanüle oder eine Infusionsleitung (beide nicht dargestellt) - je nach Anwendungszweck bzw. Art der Verabreichung - auf den Glas-Anschlußkonus 3 aufgebracht und mittels des losen Gewindeteils 5 unter Bildung einer Kegelverbindung verriegelt werden.

Die Eindrehung 4 ist von der Vorderkante des losen Gewindeteils 5 so weit entfernt, daß die Gummikappe 6 und später die Injektionskanüle oder eine Infusionsleitung ausreichend weit auf den Anschlußkonus 3 aufgeschoben werden können. Die Entfernung der Eindrehung von der Spitze des Anschlußkonus 3 beträgt vorzugsweise mehr als 5 mm.

Auf der dem Spritzenkopf gegenüberliegenden Seite ist der Spritzenzylinder 1 mit einem Kolbenstopfen 7, vorzugsweise aus Gummi, verschlossen. Dieser Kolbenstopfen weist eine Verbindungsstelle 8, vorzugsweise ein Gewinde, für die Befestigung eines Spritzenstempels 9 auf. An seinem offenen Ende ist der Spritzenzylinder 1 innen mit einer Hinterschneidung 10 versehen, in die der zylindrische Fortsatz 12 einer Griffleiste 13 mit einer komplementären Nase eingeschnappt ist, wie besonders gut aus Fig. 1b zu erkennen ist. Die Fig. 1c zeigt eine besondere Ausführungsform der sicheren Griffleistenbefestigung durch einen eingeschobenen Sicherungsring 13b, der in die Griffleiste 13 eingeschnappt ist und diese gegen Herausziehen sichert.

Bei aufgesetzter Verschlußkappe 6 und mit dem Kolbenstopfen 7 am offenen Ende des Glaszylinders 1 bei abgenommener Kolbenstange 9 sowie Spritzleiste 13 dient das erfindungsgemäße Behältnis zur langfristigen Lagerung des eingefüllten Präparates. Mit dem Behältnis kann allerdings auch im als Spritze konfektionierten Zustand gemäß Fig. 1 mit abgenommener Verschlußkappe 6 und aufgesetzter Kanüle bzw. Infusionsleitung das Präparat direkt dem Patienten zugeführt werden, indem es durch den Kolben aus dem Behältnis in die Kanüle oder Infusionsleitung verdrängt wird, wobei die Gestaltung des Behältnisses so getroffen ist, daß mit erheblichem Vorteil für Infusions- und Diagnostikzwecke die Verwendung einer Spritzenpumpe ermöglicht ist.

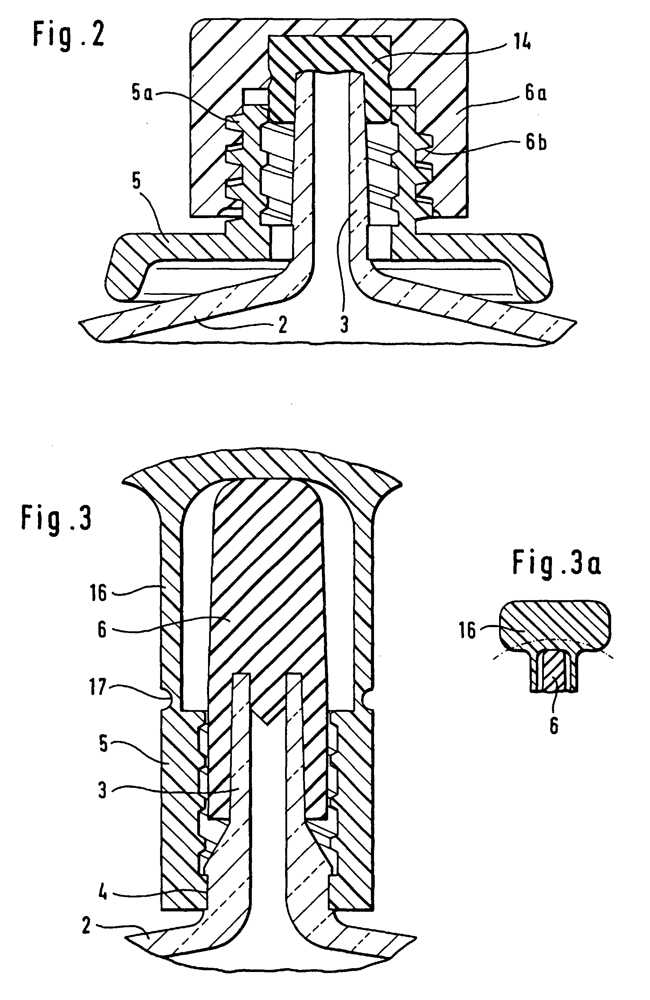

In Figur 2 ist eine zweite Ausführungsform eines Spritzenkopfes des erfindungsgemäßen Behältnisses dargestellt, der sich von dem Spritzenkopf in Figur 1 durch die Ausführung der Verschlußkappe unterscheidet. Die in Figur 2 dargestellte Verschlußkappe 6a ist mit einem Innengewinde 6b versehen und ist auf das entsprechende Außengewinde 5a des separaten Gewindeteils 5 der verriegelbaren Kegelverbindung aufgeschraubt. In die Verschlußkappe 6a ist mittig eine Gummieinlage 14 eingebracht. Diese liegt dichtend an der Mantelfläche und Stirnfläche des Anschlußkonus 3 an. Im übrigen entspricht die Ausführung nach Figur 2 derjenigen nach Fig. 1, was auch durch die Gleichheit der Bezugsziffern entsprechender Teile unterstrichen wird.

In Figur 3 ist wiederum eine besondere Ausgestaltung des Spritzenkopfes auf der Basis der Figur 1 dargestellt. Im Übergangsbereich 2 zwischen Spritzenzylinder und Anschlußkonus 3 liegt die Eindrehung 4, in die das Gewindeteil 5 der verriegelbaren Kegelverbindung eingeschnappt ist. Eine die Verschlußkappe 6 umhüllende Sicherungskappe 16 ist über eine Sollbruchstelle 17 mit dem Gewindeteil der verriegelbaren Kegelverbindung verbunden. Die Sicherungskappe 16 ist so ausgestattet, daß sie die Verschlußkappe 6 gegen unbeabsichtigtes Abziehen sichert. Zum vereinfachten Öffnen ist die Sicherungskappe 20 mit nicht dargestelten Profilierungen versehen. Die Ausbildung des Kopfteiles der Sicherungskappe 16 ist in Fig. 3a näher dargestellt. Die Bogenlinie entspricht dabei der oberen Kontur (Bruchlinie) in Fig. 3. Andere Ausführungen sind denkbar. Maßgebend ist die Grifffreundlichkeit der Ausbildung.

Der kleinste Innendurchmesser des separaten Gewindeteils 5 der verriegelbaren Kegelverbindung, inklusive Kontur der Schnappverbindung, ist größer als der größte Außendurchmesser der Verschlußkappe 6. Dies erlaubt das Montieren des separaten Gewindeteils mit Sicherungskappe 16 auch nach dem Setzen der Verschlußkappe 6.

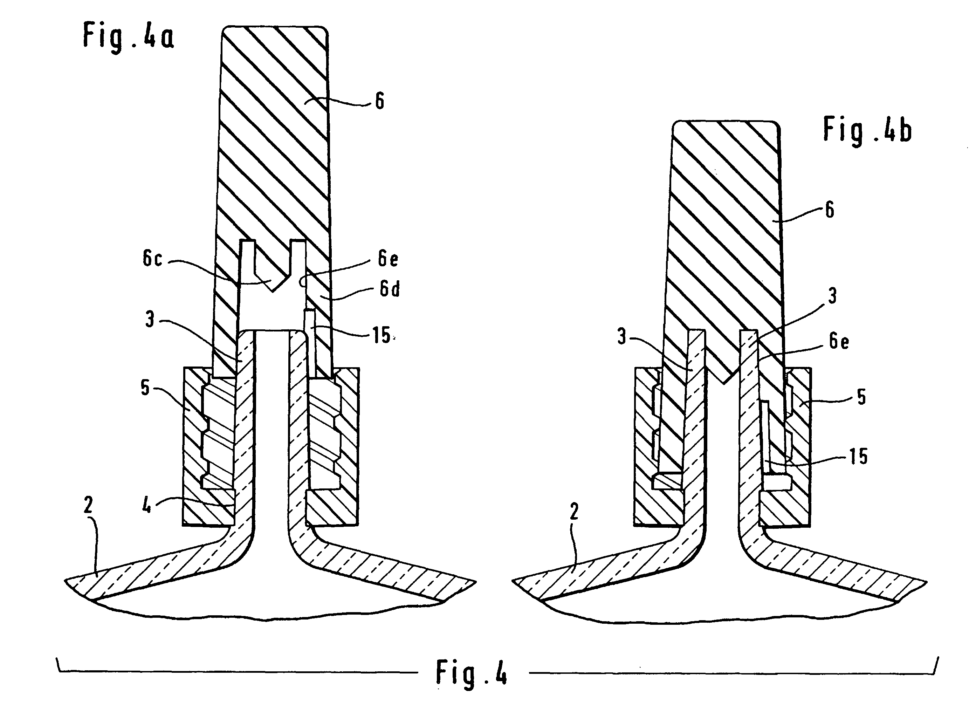

In den Fig. 4a und 4b ist ein Spritzenkopf entsprechend Fig. 1 dargestellt, jedoch mit dem Unterschied, daß die Verschlußkappe 6 im unteren Bereich des Kappenhemdes 6d auf der inneren Fläche eine oder mehrere axial verlaufende Nuten 15 aufweist.

In Figur 4a ist die Verschlußkappe 6 auf dem Anschlußkonus 3 in geöffnetem Zustand dargestellt. Der geöffnete Zustand ist dadurch gekennzeichnet, daß die Verschlußkappe 6 noch nicht vollständig auf den Anschlußkonus 3 aufgeschoben ist, so daß die Nut 15 noch eine Verbindung von der Umgebung zum Inneren der Spritze gewährleistet. Dadurch kann in der Spritze lyophilisiert werden. Figur 4b zeigt den geschlossenen Zustand. Nach dem Lyophilisieren ist die Verschlußkappe 6 vollständig auf den Anschlußkonus 3 geschoben, so daß die innere, geschlossene Kegelfläche 6e dichtend am Anschlußkonus 3 anliegt.

## Patentansprüche

1. Behältnis für Injektions-, Infusions- und Diagnostikpräparate mit einem Füllvolumen im Bereich zwischen 10 und 200ml zur langfristigen Lagerung sowie zur direkten Verabreichung der Präparate, bestehend aus einem Glaszylinder,
- bei dem die Abmessungen hinsichtlich des Verhältnisses der Gesamthöhe zum äußeren Durchmesser auf die Abmessungen einer handelsüblichen Spritze abgestellt sind (Verhältnis größer als 2,5:1),
- an dem kopfseitig ein konvergierendes Glas-Kopfteil (2) angeformt ist, das ein für die Verabreichung in eine Kanüle oder Infusionsleitung öffenbares Verschlußteil besitzt,
- der auf der anderen Seite (Fußseite) eine Kunststoff-Griffleiste (13) aufweist, die mit einem zylindrischen Fortsatz (12) an der Innenwand des Glaszylinders (1) mit einem Schnappverschluß befestigt ist, und
- der mit einem als Kolben dienenden Stopfen, der eine Verbindungsstelle für die Befestigung einer als Spritzenstempel dienenden Kolbenstange aufweist, verschlossen ist,
**dadurch gekennzeichnet, daß**
- an dem konvergierenden Glas-Kopfteil (2) ein als Spritzenkopf ausgebildeter Anschlußkonus (3) einer Kegelverbindung einstückig angeformt ist,
- zur Verriegelung der Kegelverbindung ein separates Gewindeteil (5) aus Kunststoff konzentrisch zum Anschlußkonus (3) an dessen Außenmantel befestigt ist,
- das Gewindeteil (5) zur Aufnahme einer Verschlußkappe (6), mittels der der Anschlußkonus (3) zwecks Lagerung des Behältnisinhaltes über die Kegelverbindung verschließbar ist, und zur Aufnahme der Kanüle oder Infusionsleitung über die Kegelverbindung nach vorhergehender Abnahme der Verschlußkappe zwecks direkter Verabreichung ausgebildet ist, sowie auf der der Befestigungsstelle am Außenmantel gegenüberliegenden Seite eine Sicherungskappe (16) aufweist, wobei die Verbindungsstelle zwischen dem separaten Gewindeteil und der Sicherungskappe als Sollbruchstelle (17) ausgebildet ist,
- die Griffleiste (13) so am Glaszylinder (1) befestigt ist, daß sie Abziehkräften von mindestens 100 N widersteht, indem der Glaszylinder (1) im Bereich des eingebrachten zylindrischen Fortsatzes (12) der Griffleiste (13) eine Hinterschneidung (10) und der Fortsatz eine dazu komplementäre, den Schnappverschluß bildende Hinterschneidung aufweist, und in den zylindrischen Fortsatz (12) der Griffleiste (13) ein Sicherungsring (13 b) eingeschoben ist und dort mittels einer Schnappverbindung gehaltert ist.

2. Behältnis nach Anspruch 1, **dadurch gekennzeichnet, daß** der Anschlußkonus (3) auf seiner konischen, äußeren Mantelfläche in einem Bereich, der mehr als 5 mm von seiner Spitze entfernt liegt, eine Eindrehung (4) aufweist, und daß das separate Gewindeteil (5) der verriegelbaren Kegelverbindung unter Bildung einer formschlüssigen Schnapp-Verbindung in diese Eindrehung (4) eingesetzt ist.

3. Behältnis nach Anspruch 1, **dadurch gekennzeichnet, daß** der Spritzenkopf im Übergangsbereich zwischen Anschlußkonus (3) und Spritzenzylinder (1) eine Eindrehung (4) aufweist, und daß das separate Gewindeteil (5) der verriegelbaren Kegelverbindung aus Kunststoff unter Bildung einer formschlüssigen Schnapp-Verbindung in diese Eindrehung (4) eingesetzt ist (Fig. 3).

4. Behältnis nach Anspruch 1, **dadurch gekennzeichnet, daß** auf die konische, äußere Mantelfläche des Anschlußkonus (3) in einem Bereich, der mehr als 5 mm von seiner Spitze entfernt liegt, das separate Gewindeteil (5) der verriegelbaren Kegelverbindung aus Kunststoff aufgeklebt ist.

5. Behältnis nach Anspruch 1, **dadurch gekennzeichnet, daß** der Spritzenkopf im Übergangsbereich zwischen Anschlußkonus (3) und Spritzenzylinder (1) einen Sitz aufweist, und daß das separate Gewindeteil (5) der verriegelbaren Kegelverbindung aus Kunststoff auf diesen Sitz aufgeklebt ist.

6. Behältnis nach einem der Ansprüche 1 bis 5, **dadurch gekennzeichnet, daß** das separate Gewindeteil (5) der verriegelbaren Kegelverbindung aus Kunststoff ein Außengewinde (5a) aufweist, auf das eine deckelartige Verschlußkappe (6a) mit einem Innengewinde (6b) aufgeschraubt ist, in die eine Auskleidung oder Einlage (14) aus elastischem Material eingebracht ist, die dichtend an der konischen, äußeren Mantelfläche und/oder an der Stirnfläche des Anschlußkonus (3) anliegt (Fig. 2).

7. Behältnis nach einem der Ansprüche 1 bis 5, **dadurch gekennzeichnet, daß** das separate Gewindeteil einer verriegelbaren Kegelverbindung aus Kunststoff ein Innengewinde aufweist, in das die Verschlußkappe mit einem Außengewinde eingeschraubt ist, und daß in die Verschlußkappe eine Auskleidung oder Einlage (6c) aus elastischem Material eingebracht ist, die dichtend an der konischen, äußeren Mantelfläche und/oder an der Stirnfläche des Anschlußkonus (3) anliegt (Fig. 1).

8. Behältnis nach einem der Ansprüche 1 bis 5, **dadurch gekennzeichnet, daß** die Verschlußkappe (6, 6a) aus Gummi besteht, daß die Innenkontur der Verschlußkappe eine Kontur entsprechend der Außenkontur des Anschlußkonus (3) aufweist, wobei die Innenkontur der Verschlußkappe in bezug auf die Außenkontur des Anschlußkonus vom Durchmesser her Untermaß aufweist, und daß die Verschlußkappe (6, 6a) auf den Anschlußkonus (3) aufgeschoben oder aufgedreht ist, wodurch die innere Mantelfläche der Verschlußkappe dichtend an der konischen, äußeren Mantelfläche des Anschlußkonus anliegt.

9. Behältnis nach den Ansprüchen 3 und 8 oder 5 und 8, **dadurch gekennzeichnet, daß** der kleinste Innendurchmesser des separaten Gewindeteiles (5) der verriegelbaren Kegelverbindung einschließlich Schnappverbindungsteil bzw. Klebefläche größer ist als der größte Außendurchmesser der Verschlußkappe (6, 6a).

10. Behältnis nach Anspruch 8 oder 9, **dadurch gekennzeichnet, daß** die Verschlußkappe (6) im unteren Bereich des Kappenhemdes (6 d) auf der innenliegenden Fläche eine oder mehrere axiale Nuten (15) aufweist (Fig. 4-a, b).

## Revendications

1. Récipient pour des préparations d'injection, d'infusion et de diagnostic avec un volume de remplissage de l'ordre de 10 à 200 ml pour le stockage de longue durée ainsi que pour l'administration directe des préparations, se composant d'un cylindre de verre,
- dans lequel les dimensions concernant le rapport de la hauteur totale au diamètre extérieur sont adaptées aux dimensions d'une seringue du commerce (rapport supérieur à 2,5:1),
- sur lequel une partie de tête convergente en verre (2) est formée du côté de la tête, qui possède une pièce de fermeture à ouvrir pour l'administration dans une canule ou une conduite d'infusion,
- qui présente sur l'autre (côté de pied) une poignée de saisie en plastique (13), qui est fixée par un prolongement cylindrique (12) sur la paroi intérieure du cylindre de verre (1) avec un bouchon à déclic, et
- qui est fermé avec un bouchon servant de piston, qui présente une zone d'assemblage pour la fixation d'une tige de piston servant de tampon d'injection,
**caractérisé en ce que**
- sur la partie de tête convergente en verre (2) est formé d'un seul tenant un cône de raccordement (3) d'un assemblage conique, formant une tête de seringue,
- pour le verrouillage de l'assemblage conique, une pièce filetée séparée (5) en matière plastique est fixée de façon concentrique au cône de raccordement (3) sur la surface extérieure de celui-ci,
- la pièce filetée (5) est façonnée en vue de recevoir un capuchon de fermeture (6), au moyen duquel le cône de raccordement (3) peut être fermé par l'assemblage conique pour le stockage du contenu du récipient, et en vue de recevoir la canule ou la conduite d'infusion par l'assemblage conique après l'enlèvement préalable du capuchon de fermeture pour l'administration directe, et présente un capuchon de sécurité (16) sur le côté opposé au point de fixation sur la surface extérieure, la zone d'assemblage entre la pièce filetée séparée et le capuchon de sécurité constituant une zone de moindre résistance (17),
- la poignée de saisie (13) est fixée au cylindre de verre (1) de telle manière qu'elle résiste à des efforts d'enlèvement d'au moins 100 N, du fait que le cylindre de verre (1) présente une contre-dépouille (10) dans la zone du prolongement cylindrique (12) inséré dans la poignée de saisie (13) et que le prolongement présente une contre-dépouille complémentaire à celle-ci, formant le bouchon à déclic, et une bague de fixation (13 b) est insérée dans le prolongement cylindrique (12) de la poignée de saisie (13) et y est maintenue au moyen d'un assemblage à déclic.

2. Récipient suivant la revendication 1, **caractérisé en ce que** le cône de raccordement (3) présente une portée tournée (4) sur sa surface latérale extérieure conique, dans une zone qui est située à plus de 5 mm de sa pointe, et **en ce que** la pièce filetée séparée (5) de l'assemblage conique verrouillable est introduite dans cette portée tournée (4) en formant un assemblage à déclic par emboîtement.

3. Récipient suivant la revendication 1, **caractérisé en ce que** la tête de seringue présente une portée tournée (4) dans la zone de transition entre le cône de raccordement (3) et le cylindre de seringue (1), et **en ce que** la pièce filetée séparée (5) de l'assemblage conique verrouillable en matière plastique est introduite dans cette portée tournée (4) en formant un assemblage à déclic par emboîtement (Fig. 3).

4. Récipient suivant la revendication 1, **caractérisé en ce que** la pièce filetée séparée (5) de l'assemblage conique verrouillable en matière plastique est collée sur la surface latérale extérieure conique du cône de raccordement (3), dans une zone qui est située à plus de 5 mm de sa pointe.

5. Récipient suivant la revendication 1, **caractérisé en ce que** la tête de seringue présente un siège dans la zone de transition entre le cône de raccordement (3) et le cylindre de seringue (1), et **en ce que** la pièce filetée séparée (5) de l'assemblage conique verrouillable en matière plastique est collée sur ce siège.

6. Récipient suivant l'une quelconque des revendications 1 à 5, **caractérisé en ce que** la pièce filetée séparée (5) de l'assemblage conique verrouillable en matière plastique présente un filet extérieur (5a), sur lequel est vissé un capuchon de fermeture (6a) en forme de couvercle avec un filet intérieur (6b), dans lequel est posé un revêtement ou un insert (14) en matière élastique, qui s'applique de façon étanche sur la surface latérale extérieure conique et/ou sur la face frontale du cône de raccordement (3) (Fig. 2).

7. Récipient suivant l'une quelconque des revendications 1 à 5, **caractérisé en ce que** la pièce filetée séparée d'un assemblage conique verrouillable en matière plastique présente un filet intérieur, dans lequel le capuchon de fermeture est vissé avec un filet extérieur, et **en ce qu'**un revêtement ou un insert (6c) en matière élastique est posé dans le capuchon de fermeture, en s'appliquant de façon étanche sur la surface latérale extérieure conique et/ou sur la face frontale du cône de raccordement (3) (Fig. 1).

8. Récipient suivant l'une quelconque des revendications 1 à 5, **caractérisé en ce que** le capuchon de fermeture (6, 6a) se compose de caoutchouc, **en ce que** le contour intérieur du capuchon de fermeture présente un contour correspondant au contour extérieur du cône de raccordement (3), dans lequel le contour intérieur du capuchon de fermeture présente un diamètre sous-dimensionné par rapport au contour extérieur du cône de raccordement, et **en ce que** le capuchon de fermeture, (6, 6a) est glissé ou tourné sur le cône de raccordement (3), la surface latérale intérieure du capuchon de fermeture s'appliquant ainsi de façon étanche sur la surface latérale extérieure conique du cône de raccordement.

9. Récipient suivant les revendications 3 et 8 ou 5 et 8, **caractérisé en ce que** le plus petit diamètre intérieur de la pièce filetée séparée (5) de l'assemblage conique verrouillable, y compris la pièce d'assemblage à déclic ou selon le cas la face collée, est plus grand que le plus grand diamètre extérieur du capuchon de fermeture (6, 6a).

10. Récipient suivant la revendication 8 ou 9, **caractérisé en ce que** le capuchon de fermeture (6) présente une ou plusieurs rainures axiales (15) dans la zone inférieure de la jupe du capuchon (6 d), sur la face située à l'intérieur (Fig. 4 a, b).

## Claims

1. Container for injection, infusion and diagnostic preparations, with a filling volume in the range between 10 and 200 ml, for long-term storage and direct administration of the preparations, consisting of a glass barrel
- in which the dimensions in respect of the ratio of total height to external diameter are adapted to the dimensions of a standard syringe (ratio greater than 2.5:1),
- onto which a convergent glass head part (2) is moulded at the top end, said glass head part (2) having a sealing part which can be opened for the administration into a cannula or infusion line,
- which has, at the other end (bottom end), a plastic grip (13) which, with a cylindrical extension (12), is secured on the inner wall of the glass barrel (1) by a snap-fit closure, and
- which is sealed by a stopper serving as a plunger and having a connection point for the attachment of a plunger rod serving as syringe plunger,
**characterized in that**
- an attachment cone (3) of a conical joint designed as a syringe head is moulded integrally onto the convergent glass head part (2),
- a separate threaded part (5) made of plastic is secured concentrically on the outer jacket of the attachment cone (3) in order to lock the conical joint,
- the threaded part (5) is designed to receive a sealing cap (6), by means of which the attachment cone (3) can be closed off via the conical joint for the purpose of storing the container contents, and to receive the cannula or infusion line via the conical joint after previous removal of the sealing cap for the purpose of direct administration, and it has a safety cap (16) at the end remote from the point of attachment to the outer jacket, the connection point between the separate threaded part and the safety cap being designed as a predetermined breaking point (17),
- the grip (13) is secured on the glass barrel (1) in such a way that it withstands tractive forces of at least 100 N, by means of the glass barrel (1) having an undercut (10) in the area where the cylindrical extension (12) of the grip (13) is introduced, and by means of the extension having a complementary undercut which forms the snap-fit closure, and a safety ring (13b) is pushed into the cylindrical extension (12) of the grip (13) and is retained there by means of a snap-fit connection.

2. Container according to Claim 1, **characterized in that** the attachment cone (3) has a groove (4) on its conical outer jacket surface in an area located more than 5 mm from its tip, and **in that** the separate threaded part (5) of the lockable conical joint is fitted into this groove (4) to form a positive snap-fit connection.

3. Container according to Claim 1, **characterized in that** the syringe head has a groove (4) in the transition area between attachment cone (3) and syringe barrel (1), and **in that** the separate threaded part (5) of the lockable conical joint made of plastic is fitted into this groove (4) to form a positive snap-fit connection (Fig. 3).

4. Container according to Claim 1, **characterized in that** the separate threaded part (5) of the lockable conical joint made of plastic is glued onto the conical outer jacket surface of the attachment cone (3) in an area located more than 5 mm from its tip.

5. Container according to Claim 1, **characterized in that** the syringe head has a seat in the transition area between attachment cone (3) and syringe barrel (1), and **in that** the separate threaded part (5) of the lockable conical joint made of plastic is glued onto this seat.

6. Container according to one of Claims 1 to 5, **characterized in that** the separate threaded part (5) of the lockable conical joint made of plastic has an external thread (5a) onto which a lid-like sealing cap (6a) with an internal thread (6b) is screwed, into which lid-like sealing cap (6a) a lining or insert (14) made of elastic material is introduced which bears sealingly on the conical outer jacket surface and/or on the end face of the attachment cone (3) (Fig. 2).

7. Container according to one of Claims 1 to 5, **characterized in that** the separate threaded part of a lockable conical joint made of plastic has an internal thread into which the sealing cap is screwed with an external thread, and **in that** there is introduced, into the sealing cap, a lining or insert (6c) made of elastic material which bears sealingly on the conical outer jacket surface and/or on the end face of the attachment cone (3) (Fig. 1).

8. Container according to one of Claims 1 to 5, **characterized in that** the sealing cap (6, 6a) is made of rubber, **in that** the inner contour of the sealing cap has a contour corresponding to the outer contour of the attachment cone (3), the inner contour of the sealing cap having a diameter undersized in relation to the outer contour of the attachment cone, and **in that** the sealing cap (6, 6a) is pushed or twisted onto the attachment cone (3), by which means the inner jacket surface of the sealing cap bears sealingly on the conical outer jacket surface of the attachment cone.

9. Container according to Claims 3 and 8 or 5 and 8, **characterized in that** the smallest internal diameter of the separate threaded part (5) of the lockable conical joint, including snap-fit connection part or adhesive surface, is greater than the greatest external diameter of the sealing cap (6, 6a).

10. Container according to Claim 8 or 9, **characterized in that** the sealing cap (6) has one or more axial grooves (15) on the inner surface in the lower area of the cap skirt (6d) (Fig. 4a, b).
